# EUROPEAN PATENT APPLICATION

(11) **EP 4 834 939 A1**
(43) Date of publication of application: **04.11.2026**
(21) Application number: 24911539.5
(22) Date of filing: 27.12.2024
(51) Int. Cl.: C07K 16/28, C07K 16/46, C12N 15/13, C12N 15/62, A61K 39/395, A61P 35/00, A61P 35/02, A61P 35/04

(54) **ANTI-CD16A NANOBODY, AND PREPARATION METHOD THEREFOR AND USE THEREOF**

(30) Priority: 27.12.2023 CN 202311825598
(71) Applicant: Persongen Biotherapeutics (Suzhou) Co., Ltd., Suzhou, Jiangsu 215123 (CN)
(72) Inventor: YANG, Lin, Suzhou, Jiangsu 215123 (CN); YOU, Fengtao, Suzhou, Jiangsu 215123 (CN); ZOU, Wenjun, Suzhou, Jiangsu 215123 (CN)
(74) Representative: dompatent
(86) International application number: PCT/CN2024/143360
(87) International publication number: WO 2025/140623

(57) **Abstract**

Provided are an anti-CD16A nanobody, and a preparation method therefor and the use thereof. Specifically, the method comprises: immunizing alpaca with a CD16A recombinant protein to obtain a high-quality immune library, and screening the immune library by means of using a phage display technique, to obtain a nanobody with high specificity for binding to CD16A. Further provided are a corresponding anti-CD16A antibody, chimeric antigen receptor, fusion protein and recombinant protein, an encoding nucleic acid, an expression vector, a host cell and a CAR thereof, etc. The anti-CD16A nanobody has the advantages of high affinity, simple structure and easy preparation, and has application prospects in the fields of tumor treatment, immunological detection, etc.

## Description

### TECHNICAL FIELD

The present invention relates to the field of biomedicine, and specifically, it relates to an anti-CD16A nanobody and a preparation method therefor and use thereof.

### BACKGROUND

CD16, also known as FcγRIII, is a single-pass transmembrane receptor widely expressed on the surface of natural killer cells, neutrophils, monocytes, macrophages, and some T cells. CD16 has two highly similar but distinct variants, namely FcγRIIIa (CD16a) and FcγRIIIb (CD16b), which participate in signal transduction of immune cells, trigger the lysis of target cells by NK cells, and are molecules of the immunoglobulin superfamily involved in antibody-dependent cell-mediated cytotoxicity (ADCC).

CD16 is a type III Fcγ receptor, and in humans it exists in two different forms: FcγRIIIa (CD16a) and FcγRIIIb (CD16b), which share 96% sequence identity in the extracellular immunoglobulin-binding region. FcγRIIIa is expressed as a transmembrane receptor on mast cells, macrophages, and natural killer cells, whereas FcγRIIIb is expressed only on neutrophils. FcγRIIIb is the only Fc receptor anchored to the cell membrane via a glycosylphosphatidylinositol (GPI) linker and plays an important role in triggering calcium flux signals and neutrophil degranulation. Together, FcγRIIIa and FcγRIIIb can activate degranulation, phagocytosis, and oxidative burst, thereby enabling neutrophils to clear opsonized pathogens.

CD16 binds to the Fc moiety of IgG antibodies and then activates antibody-dependent cell-mediated cytotoxicity (ADCC) in NK cells. CD16 is an essential mediator for the ADCC process in human monocytes. After binding to ligands such as the conserved moiety of IgG antibodies on human NK cells, CD16 induces gene transcription of surface activation molecules such as IL-2R (CD25) and inflammatory cytokines such as IFN-γ and TNF.

CD16 plays an important role in the early activation of natural killer (NK) cells after vaccination. In normal healthy individuals, cross-linking of immune complexes with CD16 (FcγRIII) induces antibody-dependent cell-mediated cytotoxicity (ADCC) in NK cells, through which cancer cells can also be targeted by immunotherapy. CD16-positive T cells have been found in patients with chronic viral infections or after organ transplantation, as well as in patients with severe viral infections. CD16 expression enables antibody-mediated degranulation, thereby achieving T-cell receptor-independent cytotoxicity. In severe COVID-19 patients, CD16-positive T cells may lead to exacerbated cytotoxicity, promote microvascular endothelial cell injury, and contribute to disease severity. Due to its expression on neutrophils, CD16 represents a possible target for cancer immunotherapy.

However, there is currently a lack of anti-CD16A nanobodies with high affinity, high specificity, and high biological activity. Therefore, there is an urgent need in the art to develop anti-CD16A nanobodies with high affinity, high specificity, and high biological activity.

### SUMMARY OF THE INVENTION

The object of the present invention is to provide anti-CD16A nanobodies with high affinity, high specificity, and high biological activity.

In the first aspect of the present invention, it provides an anti-CD16A nanobody, wherein the anti-CD16A nanobody has one or more complementarity determining regions (CDRs) selected from the group consisting of:
(1) CDR1 as shown in SEQ ID NO: 7, CDR2 as shown in SEQ ID NO: 8, and CDR3 as shown in SEQ ID NO: 9;
(2) CDR1 as shown in SEQ ID NO: 10, CDR2 as shown in SEQ ID NO: 11, and CDR3 as shown in SEQ ID NO: 12;
(3) CDR1 as shown in SEQ ID NO: 10, CDR2 as shown in SEQ ID NO: 13, and CDR3 as shown in SEQ ID NO: 14;
(4) CDR1 as shown in SEQ ID NO: 15, CDR2 as shown in SEQ ID NO: 16, and CDR3 as shown in SEQ ID NO: 17;
(5) CDR1 as shown in SEQ ID NO: 18, CDR2 as shown in SEQ ID NO: 19, and CDR3 as shown in SEQ ID NO: 20;
(6) CDR1 as shown in SEQ ID NO: 21, CDR2 as shown in SEQ ID NO: 22, and CDR3 as shown in SEQ ID NO: 23.

In another preferred embodiment, the CDR1, CDR2 and CDR3 are separated by framework regions FR1, FR2, FR3 and FR4 of the VHH chain.

In another preferred embodiment, the amino acid sequence of the VHH chain of the anti-CD16A nanobody is as shown in any one of SEQ ID NOs: 1-6.

In another preferred embodiment, the VHH chain of the anti-CD16A nanobody comprises an amino acid sequence having at least 80%, preferably at least 90%, more preferably at least 95%, even more preferably at least 99% sequence identity to any one of the amino acid sequences of SEQ ID NOs: 1-6.

In another preferred embodiment, the amino acid sequence of the VHH chain of the anti-CD16A nanobody comprises one or more amino acid substitutions, preferably conservative amino acid substitutions, compared to any one of the amino acid sequences of SEQ ID NOs: 1-6.

In another preferred embodiment, any of the above amino acid sequences optionally further comprises a derivative sequence obtained by addition, deletion, modification and/or substitution of at least one (e.g., 1-3, preferably 1-2, more preferably 1) amino acid, while retaining the ability to specifically bind to CD16A.

In another preferred embodiment, the anti-CD16A nanobody comprises a humanized antibody, an animal-derived antibody, or a chimeric antibody.

In the second aspect of the present invention, it provides an anti-CD16A multivalent antibody or an antibody directed against multiple epitopes, wherein the multivalent antibody or antibody directed against multiple epitopes comprises at least one antibody element directed against a CD16A epitope, and the antibody element is the anti-CD16A nanobody of the first aspect of the present invetion.

In another preferred embodiment, the anti-CD16A multivalent antibody or antibody directed against multiple epitopes comprises one or more anti-CD16A nanobodies.

In another preferred embodiment, the anti-CD16A multivalent antibody comprises a monomer, a bivalent body (divalent antibody), a tetravalent body (tetravalent antibody), and/or a multivalent body (multivalent antibody).

In another preferred embodiment, the anti-CD16A multivalent antibody comprises one or more VHH chains having an amino acid sequence as shown in any one of SEQ ID NOs: 1-6.

In another preferred embodiment, the anti-CD16A multivalent antibody comprises two VHH chains having amino acid sequences as shown in SEQ ID NOs: 1-6.

In another preferred embodiment, the anti-CD16A nanobody comprises a humanized antibody, an animal-derived antibody, or a chimeric antibody.

In the third aspect of the present invention, it provides a recombinant protein, wherein the recombinant protein has:
(i) the anti-CD16A nanobody of the first aspect of the present invention, or the anti-CD16A multivalent antibody or antibody directed against multiple epitopes of the second aspect of the present invention; and
(ii) optionally a tag sequence that assists expression and/or purification.

In another preferred embodiment, the tag sequence comprises a Flag tag, Fc tag, HA tag, or His tag.

In another preferred embodiment, the tag sequence is a His tag.

In another preferred embodiment, the recombinant protein specifically binds to CD16A protein.

In the fourth aspect of the present invention, it provides an antibody-drug conjugate, wherein the antibody-drug conjugate comprises:
(a) the nanobody of the first aspect of the present invention, the multivalent antibody or antibody directed against multiple epitopes of the second aspect of the present invention, or the recombinant protein of the third aspect of the present invention; and
(b) a conjugated moiety conjugated to the antibody moiety, wherein the conjugated moiety is selected from the group consisting of: detectable labels, drugs, toxins, cytokines, radionuclides, enzymes, and combinations thereof.

In another preferred embodiment, the antibody moiety and the conjugated moiety are conjugated via a chemical bond or a linker.

In the fifth aspect of the present invention, it provides a chimeric antigen receptor (CAR), wherein the CAR comprises an extracellular domain, and the extracellular domain comprises the anti-CD16A nanobody of the first aspect of the present invention, or the anti-CD16A multivalent antibody of the second aspect of the present invention.

In another preferred embodiment, the extracellular domain further comprises a signal peptide.

In another preferred embodiment, the extracellular domain further comprises other exogenous proteins.

In another preferred embodiment, the CAR has the structure of Formula (I):

L-Nb-H-TM-C-CD3ζ (I)

wherein,
"-" is a linker peptide or a peptide bond;
L is absent or a signal peptide sequence;
Nb is an antigen-binding domain that specifically binds to CD16A;
H is absent or a hinge region;
TM is a transmembrane domain;
C is a co-stimulatory signaling domain;
CD3ζ is a cytoplasmic signaling sequence derived from CD3ζ (comprising wild-type or mutants/modified forms thereof).

In another preferred embodiment, the L is a signal peptide selected from the group consisting of proteins: CD28, 4-1BB, GM-CSF, CD3, CD8a, and combinations thereof.

In another preferred embodiment, the sequence of the Nb is as shown in any one of SEQ ID NOs: 1-6.

In another preferred embodiment, the H is a hinge region selected from the group consisting of proteins: CD8, CD28, CD137, IgG, and combinations thereof.

In another preferred embodiment, the C is a co-stimulatory domain selected from the group consisting of proteins: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, and mutants/modified forms thereof, and combinations thereof.

In the sixth aspect of the present invention, it provides a polynucleotide, wherein the polynucleotide encodes a protein selected from the group consisting of: the nanobody of the first aspect of the present invention, the multivalent antibody or antibody directed against multiple epitopes of the second aspect of the present invention, the recombinant protein of the third aspect of the present invention, and the CAR of the fifth aspect of the present invention.

In the seventh aspect of the present invention, it provides an expression vector, wherein the expression vector comprises the polynucleotide of the sixth aspect of the present invention.

In another preferred embodiment, the expression vector is selected from the group consisting of: DNA, RNA, viral vectors, plasmids, transposons, other gene transfer systems, and combinations thereof.

In another preferred embodiment, the expression vector is a lentiviral vector.

In the eighth aspect of the present invention, it provides a host cell, wherein the host cell comprises the expression vector of the seventh aspect of the present invention, or has integrated into its genome the polynucleotide of the sixth aspect of the present invention, or expresses the nanobody of the first aspect of the present invention, the multivalent antibody or antibody directed against multiple epitopes of the second aspect of the present invention, the recombinant protein of the third aspect of the present invention, or the CAR of the fifth aspect of the present invention.

In another preferred embodiment, the cell is an isolated cell, and/or the cell is a genetically engineered cell.

In another preferred embodiment, the cell is a mammalian cell.

In another preferred embodiment, the cell is a T cell.

In another preferred embodiment, the host cell is an engineered immune cell.

In another preferred embodiment, the engineered immune cell is selected from the group consisting of:
(i) chimeric antigen receptor αβ T cells (CAR-T cells);
(ii) chimeric antigen receptor γδ T cells (CAR-T cells);
(iii) chimeric antigen receptor NKT cells (CAR-NKT cells);
(iv) chimeric antigen receptor NK cells (CAR-NK cells);
(v) chimeric antigen receptor macrophages.

In another preferred embodiment, the engineered immune cells comprise autologous or allogeneic αβ T cells, γδ T cells, NKT cells, NK cells, and combinations thereof.

In another preferred embodiment, the engineered immune cells are CAR-T cells.

In the ninth aspect of the present invention, it provides a formulation, wherein the formulation comprises the nanobody of the first aspect of the present invention, the multivalent antibody or antibody directed against multiple epitopes of the second aspect of the present invention, the recombinant protein of the third aspect of the present invention, the antibody-drug conjugate of the fourth aspect of the present invention, the CAR of the fifth aspect of the present invention, or the vector of the seventh aspect of the present invention, or immune cells expressing the CAR of the fifth aspect of the present invention, and a pharmaceutically acceptable carrier, diluent or excipient.

In the tenth aspect of the present invention, it provides a pharmaceutical composition, wherein the pharmaceutical composition comprises:
(i) the nanobody of the first aspect of the present invention, the multivalent antibody or antibody directed against multiple epitopes of the second aspect of the present invention, the recombinant protein of the third aspect of the present invention, the antibody-drug conjugate of the fourth aspect of the present invention, or the host cell of the eighth aspect of the present invention; and
(ii) a pharmaceutically acceptable carrier.

In another preferred embodiment, the pharmaceutical composition further comprises other therapeutic agents for diseases.

In another preferred embodiment, the pharmaceutical composition is used in the preparation of a medicament for the prevention and/or treatment of CD16A-associated diseases or disorders.

In another preferred embodiment, the CD16A-associated diseases or disorders is selected from the group consisting of: hematological tumors, solid tumors, and combinations thereof.

In another preferred embodiment, the hematological tumor is selected from the group consisting of: T-cell tumors, B-cell tumors, and combinations thereof.

In another preferred embodiment, the solid tumor is selected from the group consisting of: pancreatic cancer, colorectal cancer, lung cancer, gastric cancer, breast cancer, ovarian cancer, and combinations thereof.

In the eleventh aspect of the present invention, it provides a kit, wherein the kit comprises the polynucleotide of the sixth aspect of the present invention, the CAR of the fifth aspect of the present invention, or the vector of the seventh aspect of the present invention.

In another preferred embodiment, the kit is used for preparing immune cells expressing the CAR of the fifth aspect of the present invention.

In the twelfth aspect of the present invention, it provides use of the nanobody of the first aspect of the present invention, the multivalent antibody or antibody directed against multiple epitopes of the second aspect of the present invention, the recombinant protein of the third aspect of the present invention, the antibody-drug conjugate of the fourth aspect of the present invention, or immune cells expressing the CAR of the fifth aspect of the present invention, in the preparation of a medicament for the prevention and/or treatment of CD16A-associated cancer or disease.

In the thirteenth aspect of the present invention, it provides a method for treating a disease, wherein the method comprises administering to a subject in need thereof: the nanobody of the first aspect of the present invention, the multivalent antibody or antibody directed against multiple epitopes of the second aspect of the present invention, the recombinant protein of the third aspect of the present invention, the antibody-drug conjugate of the fourth aspect of the present invention, or immune cells expressing the CAR of the fifth aspect of the present invention.

In another preferred embodiment, the disease is a tumor positive for CD16A expression.

In another preferred embodiment, the subject comprises a mammal, such as a human.

It should be understood that within the scope of the present invention, the above technical features of the present invention and the technical features specifically described below (e.g., in the Examples) can be combined with each other to constitute new or preferred technical solutions. Due to space limitations, they are not repeated here.

### DESCRIPTION OF DRAWINGS

Figure 1 shows the SDS-PAGE detection result of the CD16A recombinant protein.
Figure 2 shows the amplification result of VHH fragments.
Figure 3 shows the diversity analysis of the phage display library.
Figure 4 shows the phage ELISA detection result of the fourth-round enriched products.
Figure 5 shows the FACS detection result of the target antibody expression supernatants.
Figure 6 shows the FACS binding validation results of the candidate antibodies; PC is positive control, NC is negative control.
Figure 7 shows the affinity detection results of antibodies 9-F05, 9-C05 and 9-G05.

### DETAILED DESCRIPTION OF THE INVENTION

After extensive and intensive research, the present inventors, through a large amount of screening, unexpectedly obtained for the first time anti-CD16A nanobodies with high affinity and high specificity. Specifically, the present invention uses CD16A recombinant protein to immunize alpacas, thereby obtaining a high-quality immune library. Phage display technology was used to screen the immune library, and a class of specific nanobodies against CD16A was obtained. The anti-CD16A nanobodies of the present invention have high affinity, simple structure, and are easy to prepare, and have broad application prospects in the fields of tumor therapy, immunoassay, and the like. On this basis, the present invention was completed.

### Terms

In order to facilitate understanding of the present disclosure, certain terms are first defined. As used in this application, unless otherwise expressly specified herein, each of the following terms shall have the meaning set forth below. Other definitions are set forth throughout the application.

The term "about" may refer to a value or composition within an acceptable error range for a particular value or composition as determined by one of ordinary skill in the art, which will depend in part on how the value or composition is measured or determined. For example, as used herein, the expression "about 100" comprises 99 and 101 and all values in between (e.g., 99.1, 99.2, 99.3, 99.4, etc.).

As used herein, the term "comprising" or "including" may be open-ended, semi-closed, or closed. In other words, the term also comprises "consisting essentially of" or "consisting of".

Sequence identity is determined by comparing two aligned sequences along a predetermined comparison window (which may be 50%, 60%, 70%, 80%, 90%, 95% or 100% of the length of the reference nucleotide sequence or protein) and determining the number of positions where identical residues occur. Typically, this is expressed as a percentage. Measurement of sequence identity for nucleotide sequences is well known to those skilled in the art.

### Antibodies

As used herein, the term "antibody" refers to an immunoglobulin, which is a tetrapeptide chain structure composed of two identical heavy chains and two identical light chains connected by interchain disulfide bonds.

As used herein, the terms "single domain antibody (VHH)" and "nanobody" have the same meaning and refer to cloning the variable region of the heavy chain of an antibody to construct a single domain antibody (VHH) consisting only of one heavy chain variable region, which is the smallest antigen-binding fragment with complete function. Typically, after obtaining an antibody that naturally lacks light chains and the heavy chain constant region 1 (CH1), the variable region of the heavy chain is cloned to construct a single domain antibody consisting only of one heavy chain variable region.

The term "antigen-binding fragment of an antibody" (or simply "antibody fragment") refers to one or more fragments of an antibody that retain the ability to specifically bind to an antigen (e.g., CD16A). It has been shown that fragments of full-length antibodies can be used to perform the antigen-binding function of antibodies. Examples of binding fragments encompassed within the term "antigen-binding fragment of an antibody" comprise:
(i) Fab fragments, monovalent fragments composed of VL, VH, CL and CH1 domains;
(ii) F(ab')₂ fragments, bivalent fragments comprising two Fab fragments linked by a disulfide bridge at the hinge region;
(iii) Fd fragments composed of VH and CH1 domains;
(iv) Fv fragments composed of VH and VL domains of a single arm of an antibody.

Fv antibodies comprise the heavy chain variable region and light chain variable region of an antibody, but no constant region, and are the smallest antibody fragments with all antigen-binding sites. Generally, Fv antibodies also comprise a polypeptide linker between the VH and VL domains and are capable of forming a structure required for antigen binding.

The term "CDR" refers to one of the six hypervariable regions within the variable domains of an antibody that primarily contribute to antigen binding. One of the most commonly used definitions of the six CDRs is provided by Kabat E.A. et al., (1991) Sequences of proteins of immunological interest. NIH Publication, No. 91-3242.

The term "epitope" or "antigenic determinant" refers to a site on an antigen to which an immunoglobulin or antibody specifically binds (e.g., a specific site on the CD16A molecule). Epitopes typically comprise at least 3, 4, 5, 6, 7, 8, 9, 10, 11, 12, 13, 14, or 15 consecutive or non-consecutive amino acids in a unique spatial conformation.

The terms "specific binding," "selective binding," "selectively binds," and "specifically binds" refer to the binding of an antibody to an epitope on a predetermined antigen. Typically, the antibody binds with an affinity (KD) of approximately less than 10⁻⁷ M, for example, approximately less than 10⁻⁸ M, 10⁻⁹ M, or 10⁻¹⁰ M or less.

As used herein, the term "antigenic determinant" refers to a discontinuous three-dimensional spatial site on an antigen that is recognized by the antibodies or antigen-binding fragments of the present invention.

The present invention comprises not only intact antibodies, but also fragments of antibodies with immunological activity or fusion proteins formed by antibodies and other sequences. Accordingly, the present invention also comprises fragments, derivatives, and analogs of the antibodies.

In the present invention, the antibodies of the present invention also comprise conservative variants thereof, which refer to polypeptides formed by replacing at most 10, preferably at most 8, more preferably at most 5, and most preferably at most 3 amino acids with amino acids of similar or analogous properties compared to the amino acid sequence of the antibodies of the present invention. These conservative variant polypeptides are preferably produced by amino acid substitutions according to Table A below.

**Table A**

| Original Residue | Representative Substitutions | Preferred Substitutions |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD16A Nanobody

As used herein, the term "CD16A" generally refers to natural or recombinant human CD16A, as well as non-human homologs of human CD16A.

As used herein, the terms "nanobody of the present invention," "single-domain antibody of the present invention," and "anti-CD16A nanobody of the present invention" are used interchangeably and all refer to the nanobodies of the present invention that have high specificity and high affinity against CD16A.

Compared with traditional monoclonal antibodies, the nanobodies of the present invention have small molecular weight, strong penetrating ability, high sensitivity, high specificity, and good stability; after entering the body, they can efficiently penetrate cells to rapidly capture antigens to achieve therapeutic purposes.

In the present invention, the anti-CD16A nanobody comprises a monomer, a divalent antibody, a tetravalent antibody, and/or a multivalent antibody.

In a preferred embodiment of the present invention, the amino acid sequence of the VHH chain of the anti-CD16A nanobody is selected from one or more of SEQ ID NOs: 1-6. The preferred nanobody names, VHH sequence numbers, and corresponding CDR region sequence numbers of the present invention are shown in Table 1:

**Table 1**

| Antibody Name | CDR1 | CDR2 | CDR3 | VHH Chain Amino Acid Sequence No. |
|---|---|---|---|---|
| 9-G05 | 7 | 8 | 9 | 1 |
| 9-F05 | 10 | 11 | 12 | 2 |
| 9-C05 | 10 | 13 | 14 | 3 |
| 9-H03 | 15 | 16 | 17 | 4 |
| 7-F11 | 18 | 19 | 20 | 5 |
| 7-H2 | 21 | 22 | 23 | 6 |

| | | | | |
|---|---|---|---|---|
| Note: The numerical values in the table indicate sequence numbers, i.e., "1" means "SEQ ID NO: 1"; identical sequences share the same number, e.g., CDR1 of 9-F05 and CDR1 of 9-C05 are the same sequence. | | | | |

### Antibody-Drug Conjugates (ADC)

The present invention also provides antibody-drug conjugates (ADCs) based on the nanobodies of the present invention.

Typically, the antibody-drug conjugate comprises the antibody and an effector molecule, wherein the antibody is conjugated to the effector molecule, preferably via chemical conjugation. The effector molecule is preferably a therapeutically active drug. In addition, the effector molecule may be one or more of a toxic protein, a chemotherapeutic drug, a small molecule drug, or a radionuclide.

The antibody of the present invention and the effector molecule may be conjugated via a coupling agent. Examples of the coupling agent may be any one or more of non-selective coupling agents, coupling agents utilizing carboxyl groups, peptide linkers, or coupling agents utilizing disulfide bonds. Non-selective coupling agents refer to compounds that form covalent bonds between the effector molecule and the antibody, such as glutaraldehyde. Coupling agents utilizing carboxyl groups may be any one or more of cis-aconitic anhydride-type coupling agents (e.g., cis-aconitic anhydride) or acylhydrazone-type coupling agents (coupling site at acylhydrazone).

Certain residues on the antibody (e.g., Cys or Lys, etc.) are used to attach to various functional groups, comprising imaging reagents (e.g., chromophores and fluorophores), diagnostic reagents (e.g., MRI contrast agents and radioisotopes), stabilizers (e.g., ethylene glycol polymers), and therapeutic agents. Antibodies can be conjugated to functional agents to form antibody-functional agent conjugates. Functional agents (e.g., drugs, detection reagents, stabilizers) are conjugated (covalently attached) to the antibody. The functional agent may be attached directly or indirectly via a linker to the antibody.

Antibodies can be conjugated to drugs to form antibody-drug conjugates (ADCs). Typically, an ADC comprises a linker between the drug and the antibody. The linker may be a cleavable or non-cleavable linker. Cleavable linkers are typically readily cleaved in the intracellular environment, for example, the linker is cleaved at the target site, thereby releasing the drug from the antibody. Suitable cleavable linkers comprise, for example, enzyme-cleavable linkers, comprising peptide-based linkers that are cleavable by intracellular proteases (e.g., lysosomal proteases or endosomal proteases), or sugar linkers, e.g., glucuronide-containing linkers that can be cleaved by glucuronidase. Peptidyl linkers may comprise, for example, dipeptides such as valine-citrulline, phenylalanine-lysine, or valine-alanine. Other suitable cleavable linkers comprise, for example, pH-sensitive linkers (e.g., linkers that hydrolyze at pH less than 5.5, such as hydrazone linkers) and linkers that degrade under reducing conditions (e.g., disulfide linkers). Non-cleavable linkers typically release the drug under conditions where the antibody is hydrolyzed by proteases.

Before attachment to the antibody, the linker has active reactive groups that can react with certain amino acid residues, and attachment is achieved through these reactive groups. Sulfhydryl-specific reactive groups are preferred and include, for example: maleimides, haloacetamides (e.g., iodo, bromo, or chloro); haloesters (e.g., iodo, bromo, or chloro); halomethyl ketones (e.g., iodo, bromo, or chloro), benzyl halides (e.g., iodo, bromo, or chloro); vinyl sulfones, pyridyl disulfides; mercury derivatives such as 3,6-di-(mercurimethyl)dioxane, with counterions acetate, chloride, or nitrate; and polymethylene dimethyl thioether sulfonates. The linker may comprise, for example, maleimide attached to the antibody via succinimide.

The drug may be any cytotoxic, cytostatic, or immunosuppressive agent. In embodiments, the linker connects the antibody and the drug, and the drug has a functional group capable of bonding with the linker. For example, the drug may have an amino, carboxyl, sulfhydryl, hydroxyl, or ketone group capable of bonding with the linker. In cases where the drug is directly attached to the linker, the drug has a reactive active group before attachment to the antibody.

Useful classes of drugs comprise, for example, anti-tubulin agents, DNA minor groove binding agents, DNA replication inhibitors, alkylating agents, antibiotics, folate antagonists, antimetabolites, chemosensitizers, topoisomerase inhibitors, vinca alkaloids, and the like. In the present invention, the drug-linker may be used to form an ADC in a single step. In other embodiments, a bifunctional linker compound may be used to form an ADC in two or more steps. For example, a cysteine residue reacts with the reactive moiety of the linker in a first step, and then in a subsequent step, a functional group on the linker reacts with the drug to form the ADC.

Generally, the functional groups on the linker are selected to facilitate specific reaction with suitable reactive active groups on the drug moiety. As non-limiting examples, azide-based moieties may be used to specifically react with reactive alkynyl groups on the drug moiety. The drug is covalently attached to the linker via a 1,3-dipolar cycloaddition between the azide and the alkyne. Other useful functional groups comprise, for example, ketones and aldehydes (suitable for reaction with hydrazides and alkoxyamines), phosphines (suitable for reaction with azides), isocyanates and isothiocyanates (suitable for reaction with amines and alcohols), and activated esters, such as N-hydroxysuccinimide esters (suitable for reaction with amines and alcohols). These and other conjugation strategies, such as those described in Bioconjugate Techniques, 2nd Edition (Elsevier), are well known to those skilled in the art. Those skilled in the art will appreciate that for selective reaction between the drug moiety and the linker, when a complementary pair of reactive active functional groups is chosen, each member of the complementary pair may be used on either the linker or the drug.

The present invention also provides a method for preparing an ADC, which may further comprise: combining the antibody with a drug-linker compound under conditions sufficient to form the antibody conjugate (ADC).

In certain embodiments, the method of the present invention comprises: combining the antibody with a bifunctional linker compound under conditions sufficient to form an antibody-linker conjugate. In these embodiments, the method of the present invention further comprises: combining the antibody-linker conjugate with a drug moiety under conditions sufficient to covalently attach the drug moiety to the antibody via the linker.

### Pharmaceutical Compositions

The present invention also provides a composition. In a preferred embodiment, the composition is a pharmaceutical composition, which comprises the above antibody or active fragment thereof, or fusion protein thereof, or ADC thereof, or corresponding CAR-T cells, and a pharmaceutically acceptable carrier. Generally, these substances can be formulated in non-toxic, inert, and pharmaceutically acceptable aqueous carrier media, with a pH generally about 5-8, preferably about 6-8, although the pH may vary depending on the nature of the substance being formulated and the condition to be treated. The formulated pharmaceutical composition can be administered by conventional routes, including but not limited to: intratumoral, intraperitoneal, intravenous, or topical administration.

The antibodies of the present invention may also be expressed by nucleotide sequences in cells for cell therapy, for example, the antibodies may be used in chimeric antigen receptor T-cell immunotherapy (CAR-T), etc.

The pharmaceutical composition of the present invention can be directly used to bind to CD16A protein molecules, and thus can be used for the prevention and treatment of CD16A-related diseases. In addition, other therapeutic agents may be used concurrently.

The pharmaceutical composition of the present invention comprises a safe and effective amount (e.g., 0.001-99 wt%, preferably 0.01-90 wt%, more preferably 0.1-80 wt%) of the above monoclonal antibody (or conjugate thereof) of the present invention, and a pharmaceutically acceptable carrier or excipient. Such carriers include (but are not limited to) : saline, buffer, glucose, water, glycerol, ethanol, and combinations thereof. The pharmaceutical formulation should match the mode of administration. The pharmaceutical composition of the present invention can be prepared in the form of an injectable solution, for example, by conventional methods using physiological saline or an aqueous solution containing glucose and other adjuvants. Pharmaceutical compositions such as injectable solutions should be manufactured under sterile conditions. The amount of active component administered is a therapeutically effective amount, for example, about 1 µg/kg body weight to about 5 mg/kg body weight per day. In addition, the polypeptides of the present invention may also be used together with other therapeutic agents.

When using the pharmaceutical composition, a safe and effective amount of the pharmaceutical composition is administered to a mammal, wherein the safe and effective amount is generally at least about 10 µg/kg body weight, and in most cases does not exceed about 50 mg/kg body weight, preferably the dose is about 10 µg/kg body weight to about 20 mg/kg body weight. Of course, the specific dosage should also take into account factors such as the route of administration and the patient's health condition, which are within the skill of the attending physician.

### Main advantages of the present invention comprise:

(1) The nanobodies of the present invention have high affinity binding to CD16A.
(2) The nanobodies of the present invention can specifically bind to CD16A but not to CD16B, and have high specificity.
(3) The anti-CD16A nanobodies of the present invention have small molecular weight, strong penetrating ability, and high sensitivity.
(4) The anti-CD16A nanobodies of the present invention have simple structure and are easy to prepare, and have broad application prospects in the fields of tumor therapy, immunoassay, and the like.

The present invention is further illustrated below in conjunction with specific examples. It should be understood that these examples are only for illustrating the present invention and are not intended to limit the scope of the present invention. In the following examples, experimental methods without specified conditions are generally carried out under conventional conditions, for example, as described in Sambrook et al., Molecular Cloning: A Laboratory Manual (New York: Cold Spring Harbor Laboratory Press, 1989), or according to the conditions recommended by the manufacturer. Unless otherwise indicated, percentages and fractions are by weight and weight fractions.

### Example 1: Screening of Antibodies Against CD16A

### 1.1 Preparation of CD16A Recombinant Protein

The extracellular domain sequence (Gly17-Gln208) of human CD16A (P08637-1) was retrieved from the UniProt database, a His tag was added at the C-terminus, and after optimization according to human codon preference, gene synthesis was performed and subcloned into the pcDNA3.4 vector. After verification by Sanger sequencing, plasmid extraction was performed for later use. The constructed protein eukaryotic expression vector was transiently transfected into 293F cells, and the protein expression supernatant was collected and the target protein was purified using a nickel column; SDS-PAGE was performed to detect protein purity.

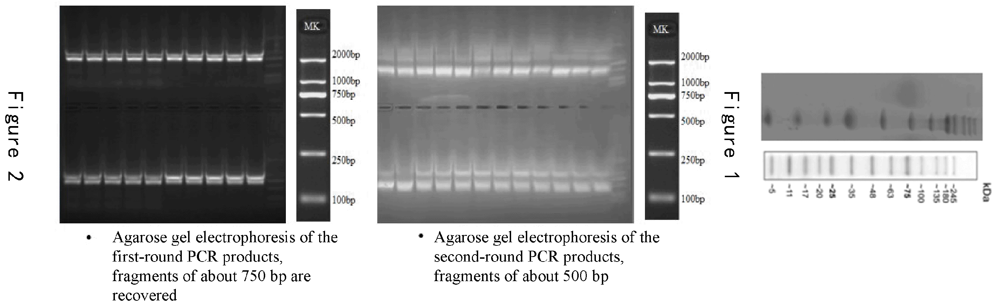

The results are shown in Figure 1. The purity of the human CD16A recombinant protein was >90%, indicating that it could be used for subsequent alpaca immunization and antibody screening.

### 1.2 Alpaca Immunization

Alpacas were immunized with the recombinant protein prepared above, with an immunization interval of 21 days. Ten days after the last immunization, peripheral blood was collected, and serum was separated for ELISA to detect the immune response.

### 1.3 Detection of Immune Titers

Serum was isolated from immunized alpacas, serially diluted according to the dilution gradients in Table 2, and ELISA was performed with antigen-precoated 96-well plates. The experimental method was as follows:
5 mL of peripheral blood was collected, and the centrifuge tube containing the blood sample was placed in a 37 °C incubator for 1 hour; then the blood sample was transferred to 4 °C overnight. The centrifuge tube containing the blood sample was centrifuged at 5000 rpm for 20 min; the upper serum layer was separated and transferred to a new sterile centrifuge tube to collect immune serum. The target recombinant protein was diluted with sterile CBS (carbonate buffer) to a final concentration of 1 µg/mL. A new 96-well ELISA plate was taken, and 100 µL/well was added, and coated overnight at 4 °C. The antigen coating solution was discarded, and the plate was washed 5 times with PBST (containing 0.05% Tween 20). 200 µL/well of 3% MPBS was added for blocking at 37 °C for 2 hours; after removing the blocking buffer, the plate was washed 5 times with PBST; 100 µL of serially diluted serum (100 µL/well) was added and incubated at room temperature for 1 hour, with PBS as control wells; the liquid in the wells was discarded, and the plate was washed 5 times with PBST; 100 µL of HRP anti-Lama IgG (H+L) antibody (1:50000 dilution) was added and incubated at room temperature for 1 hour; after discarding the liquid, the plate was washed 5 times with PBST; 100 µL/well of TMB substrate was added; incubated at room temperature in the dark for 10-15 minutes; 50 µL/well of stop solution was added; the OD450 value was read using a microplate reader.

The detection results are shown in Table 2 below.

**Table 2 Detection of serum immune titers**

| Serum Dilution Series | Negative Serum | | Third Immunization | |
|---|---|---|---|---|
| 1:1K | 0.347 | 0.335 | 2.615 | 2.559 |
| 1:2K | 0.194 | 0.181 | 2.412 | 2.379 |
| 1:4K | 0.133 | 0.144 | 2.125 | 2.096 |
| 1:8K | 0.127 | 0.147 | 1.626 | 1.627 |
| 1:16K | 0.188 | 0.118 | 1.055 | 1.124 |
| 1:32K | 0.13 | 0.107 | 0.661 | 0.648 |
| 1:64K | 0.078 | 0.081 | 0.373 | 0.363 |
| PBS | 0.072 | 0.071 | 0.069 | 0.068 |

According to the ELISA results, the immune serum could bind to the CD16A recombinant protein, and the OD values changed in a gradient manner with serial dilution of the immune serum. After the immune titer reached above 1:64K, 100 mL of peripheral blood was collected for cDNA library preparation to construct the antibody display library.

### 1.4 PBMC Isolation and Cloning of VHH Antibody Fragments

1. 100 mL of peripheral blood was collected, and PBMCs were isolated using lymphocyte separation medium.
2. RNA was extracted, and reverse transcription was performed using the PrimeScript^{™} II 1st Strand cDNA Synthesis Kit to prepare cDNA. In a 200 µL PCR tube, the reaction mixture Mix1 was prepared as shown in Table 3:

**Table 3 Preparation system for reaction mixture Mix1**

| Reagent | Amount |
|---|---|
| Oligo dT Primer (50 µM) | 8 µL |
| dNTP Mixture (each 10 mM) | 8 µL |
| Total RNA sample | 20 µg |
| RNase-Free water | make up to 80 µL |

After incubation at 65°C for 5 min, the tube was rapidly cooled on ice. The following reaction solution was prepared in the above PCR tube as shown in Table 4:

**Table 4 Preparation system for reaction solution**

| Reagent | Amount |
|---|---|
| Denatured reaction solution from above | 80 µL |
| 5×PrimeScript II Buffer | 32 µL |
| RNase Inhibitor (40 M/µL) | 4 µL |
| PrimeScript II RTase (200 M/µL) | 8 µL |
| RNase-Free water | 36 µL |

After mixing by pipetting, it was dispensed at 80 µL/tube, placed in a PCR instrument at 42°C for 1 hour, and heat-inactivated at 70°C for 15 minutes. The cDNA sample was then placed on ice or stored at -20°C for long-term storage.

### 3. Amplification of VHH fragments:

(1) The first-round PCR reaction system (50 µL/tube) was prepared as shown in Table 5: Table 5 First-round PCR reaction system

| Component | Amount |
|---|---|
| Forward primer (5 µM) | 2 µL |
| Reverse primer (10 µM) | 1 µL |
| NuHi Power mix (2×) | 25 µL |
| cDNA template | 2 µL |
| Sterile water | 20 µL |

After preparing the PCR reaction system, the PCR instrument was programmed according to the protocol in Table 6:

**Table 6 PCR program settings**

| Program | Temperature | Time | Cycles |
|---|---|---|---|
| Initial denaturation | 95°C | 10 min | |
| Denaturation | 95°C | 15 s | 25X |
| Annealing | 55°C | 30 s | |
| Extension | 68°C | 1 min | |
| Final extension | 68°C | 10 min | |

(2) Agarose gel electrophoresis of PCR products: 1% agarose gel was used for electrophoresis analysis of PCR products, and fragments of about 750 bp were separated. The PCR products were recovered using a gel extraction kit, and the concentration was measured with a NanoDrop.

(3) The second-round PCR reaction system (50 µL/tube) was prepared as shown in Table 7:

**Table 7 Second-round PCR reaction system**

| Component | Amount |
|---|---|
| 2^{nd} F primer | 2 µL |
| 2^{nd} R primer | 2 µL |
| NuHi Power mix (2×) | 25 µL |
| First-round PCR purified product | 200 ng |
| Sterile water | make up to 50 µL |

After preparing the PCR reaction system, the PCR instrument was programmed according to the protocol in Table 8:

**Table 8 PCR program settings**

| Program | Temperature | Time | Cycles |
|---|---|---|---|
| Initial denaturation | 95°C | 10 min | |
| Denaturation | 95°C | 15 s | 18X |
| Annealing | 55°C | 30 s | |
| Extension | 68°C | 1 min | |
| Final extension | 68°C | 10 min | |

(4) Agarose gel electrophoresis of the second-round PCR products: 1% agarose gel was used for electrophoresis analysis of PCR products, and VHH fragments of about 400 bp were separated. The VHH PCR products were recovered using a gel extraction kit, and the concentration was measured with a NanoDrop.

The agarose gel electrophoresis results of the PCR products are shown in Figure 2. The first-round PCR obtained bands of about 1000 bp and 750 bp, and the 750 bp fragment was recovered as the template for the second-round PCR. The second-round PCR obtained a band of about 400 bp, which was the VHH fragment. The pDisplay vector and the VHH PCR gel-purified products obtained above were digested with SfiI, respectively. The digested pDisplay vector and VHH fragments were ligated using T4 ligase, incubated at 16°C overnight, and then transformed into E. coli.

### 1.5 Construction of Phage Display Library

### 1. Construction of single-domain antibody phage display vector

The pDisplay vector and the VHH PCR gel-purified products obtained above were digested with SfiI, respectively. The digestion systems are shown in Table 9 and Table 10:

**Table 9 Digestion system**

| Component | Amount |
|---|---|
| pDisplay | 140 µg |
| 10×CutSmart^{®} Buffer | 200 µL |
| SfiI | 1200 U (60 µL) |
| Sterile water | Up to 2000 µL |

The digestion system shown in Table 8 was dispensed into 100 µL/tube and digested overnight at 50 °C on a PCR instrument.

**Table 10 Digestion system**

| Component | Amount |
|---|---|
| Second-round PCR purified product | 10 µg |
| 10×CutSmart^{®} Buffer | 20 µL |
| SfiI | 360 U (18 µL) |
| Sterile water | make up to 200 µL |

The digestion system shown in Table 9 was dispensed into 100 µL/tube and digested overnight at 50 °C on a PCR instrument. The pDisplay vector fragment was separated by 1% agarose gel electrophoresis, and the 5000 bp vector fragment was excised and gel-purified; meanwhile, the PCR digestion products were purified using a DNA fragment purification kit, and the concentration was measured with a NanoDrop. The digested pDisplay vector and VHH fragments were ligated using T4 ligase, incubated at 16 °C overnight.

### 2. Electroporation of ligation products into E. coli

The electroporation cuvettes, ligation products, and electrocompetent cells were pre-chilled on ice. The pre-chilled library ligation product was added to the electrocompetent cells and kept on ice for 1 min. 300 µL of DNA/competent cell mixture was added to each cuvette, and the cuvettes were placed on ice. Electroporation was performed at 2500 V, 5 ms. Immediately after electroporation, SOC medium pre-equilibrated to room temperature was added to resuspend the cells, and the cells were cultured at 37°C with shaking for 1 hour. 15 mL of the bacterial culture was directly used for phage rescue, and the remaining 5 mL of electroporation product was mixed with an equal volume of 50% glycerol and stored at -80 °C. In addition, 20 µL of the bacterial culture was taken and diluted with 980 µL of 2YT medium, then 100 µL of the diluted product was taken and diluted again with 900 µL of 2YT medium, and 50 µL was spread evenly on LB plates containing ampicillin and cultured overnight at 37 °C. The next day, the plates were taken out, and the number of colonies generated per ligation was counted to calculate the library size. At the same time, 20 single colonies were picked from the plates and inoculated into 2YT medium containing ampicillin, cultured with shaking at 37 °C for about 6-8 hours, and the cultures were sent for sequencing (using the universal primer M13R). 20 randomly selected single colonies were sequenced to analyze the diversity of the constructed phage display library.

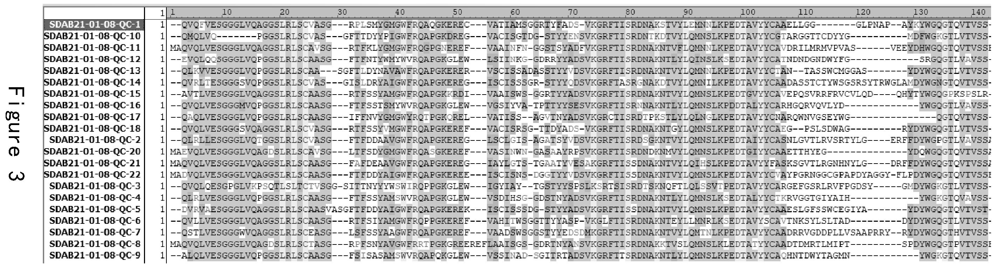

The results are shown in Figure 3. Sequencing alignment showed that the empty rate and antibody repeat rate of the phage display library were not more than 5%. The library size of the E. coli library was 5.36×10⁸.

### 1.6 Phage Library Preparation and Phage Precipitation

The electroporated transformation product was diluted with 2YT to adjust the OD₆₀₀ to about 0.2, ampicillin was added to a final concentration of 100 µg/mL, and the culture was incubated in a constant-temperature shaker at 37 °C, 225 rpm, until OD₆₀₀ reached 0.5. M13KO7 helper phage was added, mixed, and incubated at 37°C statically for 30 min, then cultured at 37 °C, 225 rpm for 1 h. The volume of M13KO7 = 10 × volume × OD₆₀₀ × 5×10⁸ / M13KO7 titer. The bacterial culture was centrifuged at 6000 rpm for 10 min, and the pellet was resuspended in 2YT-AK medium, and cultured overnight at 25 °C, 200 rpm. The bacterial culture was centrifuged at 10000 rpm for 15 min; the supernatant was transferred to a new centrifuge tube and the pellet was discarded, and 1/5 volume of PEG/NaCl was added, mixed, and placed at 4 °C for 2 h. The precipitated phage supernatant was centrifuged at 10000 rpm, 4 °C for 30 min; the supernatant was discarded, and the pellet (phage) in each 50 mL centrifuge tube was resuspended in 1 mL of sterile PBS. The resuspended phage was transferred to a 1.5 mL EP tube and centrifuged at 12000 g, 4 °C for 5 min; the supernatant was transferred to a new 1.5 mL EP tube, 250 µL of PEG/NaCl was added, mixed, and incubated at 4 °C for 10 min, centrifuged at 12000 g for 10 min, the supernatant was discarded, and the pellet was resuspended in 1 mL of PBS, centrifuged at 12000 g for 5 min, the supernatant was transferred to a new 1.5 mL tube, and centrifuged again at 12000 g for 5 min, and the supernatant was transferred to a new 1.5 mL EP tube to obtain the original phage library. 10 µL of the precipitate was mixed with 90 µL of 2YT medium (designated as 10⁻¹), and then serially diluted 10-fold to 10⁻⁹. 20 µL of the 10⁻⁷, 10⁻⁸, and 10⁻⁹ dilutions were added to 200 µL of ER2738 with OD600=0.5, mixed, and incubated at 37 °C water bath for 10 min. 100 µL of each mixture was spread on LB-AMP solid plates and cultured overnight at 37 °C. The colonies were counted the next day to determine the titer. Titer calculation: plates with 30-300 colonies were selected, the average of two plates was taken, and the number of colonies was multiplied by the dilution factor and then by 100 to obtain the titer.

### 1.7 Panning of the Phage Display Library

Panning was performed using the recombinant protein. The phage display library was incubated with the recombinant protein, and the recombinant phages bound to the target antigen were eluted with TEA and amplified. After 3-4 rounds of panning, single clones were picked for sequencing.

Approximately 6×10¹¹ pfu (150 µL) of the phage library was diluted with 1% PBSA to about 1 mL, and added to the target protein-coated ELISA plate, and incubated at 4°C for 1 h. The bound phages were eluted with 600 µL of 1×TEA for 10 min. The eluted product was transferred to a pre-blocked EP tube, and 300 µL of Tris-HCl was added for neutralization. 10 µL of the output product was added to 90 µL of 2YT medium (designated as 100), and then serially diluted 10-fold to 10⁻². 20 µL of the 101, 100, 10⁻¹, and 10⁻² dilutions were added to 200 µL of ER2738 with OD₆₀₀=0.5 (for 101, 20 µL of undiluted output was directly added to ER2738), mixed, and incubated at 37 °C water bath for 10 min. 100 µL of each mixture was spread on LB-AMP solid plates and cultured overnight at 37 °C. Colonies were counted the next day to determine the titer. Titer calculation: plates with 30-300 colonies were selected, the average of two plates was taken, and the number of colonies was multiplied by the dilution factor and then by the elution volume.

The target antigen recombinant protein was co-incubated with the single-domain antibody phage display library to enrich for phages specifically binding to the target antigen. After four rounds of enrichment, the input and output of each round were counted, and the enrichment factor for each round was calculated. The results are shown in Table 11.

**Table 11 Enrichment results for each round**

| Round | Input (PFU) | Output (PFU) | Enrichment Factor |
|---|---|---|---|
| 1 | 3.79E+13 | 8.74E+06 | 1.58E+06 |
| 2 | 1.81E+13 | 6.08E+06 | 1.75E+06 |
| 3 | 2.55E+13 | 4.21E+07 | 3.02E+05 |
| 4 | 1.29E+13 | 6.15E+07 | 1.79E+05 |

### 1.8 Phage ELISA

2YT-Amp medium was dispensed into 96-well deep-well plates, 500 µL per well. Single colonies from the output plates were picked and cultured at 37°C, 225 rpm until OD₆₀₀ reached 0.5. Wells E12 and F12 were not subjected to colony picking; only culture medium was added to them, and they were used as blank controls. Wells G12 and H12 were not subjected to colony picking; the binding of the antigen to the positive antibody was detected in them, and they were used as positive control. Meanwhile, ELISA plates were coated with antigen (1 µg/mL, 100 µL/well) in CBS at 37°C for 2 h. Another 96-well deep-well plate was filled with 500 µL of 2YT-A medium per well, and 10 µL of the culture with OD₆₀₀=0.5 was transferred to the new plate using a multichannel pipette, and cultured overnight at 37°C, 225 rpm (for sequencing). To the culture with OD₆₀₀=0.5, M13KO7 was added, mixed, and incubated at 37 °C statically for 15 min. Volume of M13KO7 = 10 × volume × OD₆₀₀ × 5×10⁸ / M13KO7 titer. The infected culture was shaken at 37 °C, 225 rpm for 45 min. The culture was centrifuged at 4000 rpm for 10 min, the supernatant discarded, and the pellet resuspended in 2YT-AK medium at 800 µL per well, placed in a shaker, and the suspension was cultured overnight at 30 °C with shaking at 210 rpm. Meanwhile, the antigen solution in the ELISA plate was discarded, and the plate was washed three times with PBST. Then it was blocked with 3% MPBS at 250 µL/well overnight at 4 °C. And an additional blank plate was blocked as BLANK. On the next day, the deep-well plate was placed in a centrifuge and centrifuged at 4000 rpm for 10 min. The milk in the ELISA plate was discarded, and the plate was washed 4 times with 200 µL PBST. 50 µL of PBST was added to each well, followed by 50 µL of the centrifuged phage supernatant, and incubated at 4 °C for 1 h. After discarding the supernatant, the plate was washed 5 times with PBST. HRP-Anti-M13 secondary antibody diluted in PBST was added (100 µL/well) and incubated at 4 °C for 45 min, then the secondary antibody was removed by washing 5 times with PBST, TMB color development at room temperature for 10 min, stopped with HCl, and the absorbance was read. Clones with S/N ratio >6 were selected, and the corresponding bacterial glycerol stocks were sent for sequencing to analyze antibody sequences. After panning of the phage display library, different antibody sequences were obtained, and expression vectors were constructed for validation.

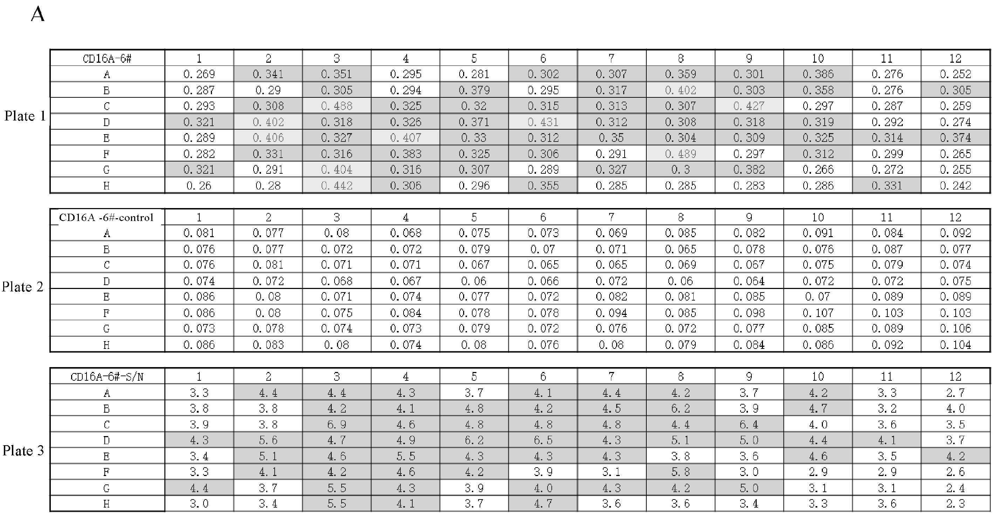

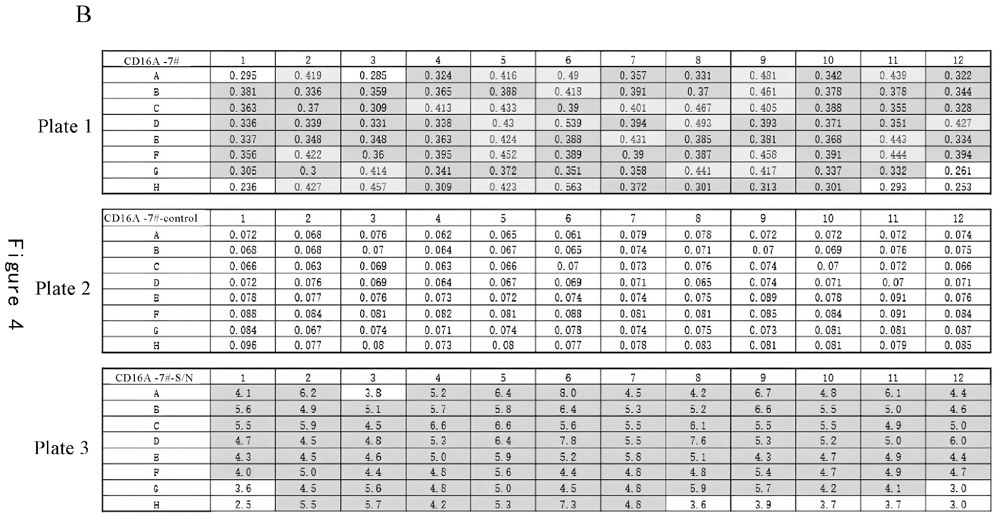

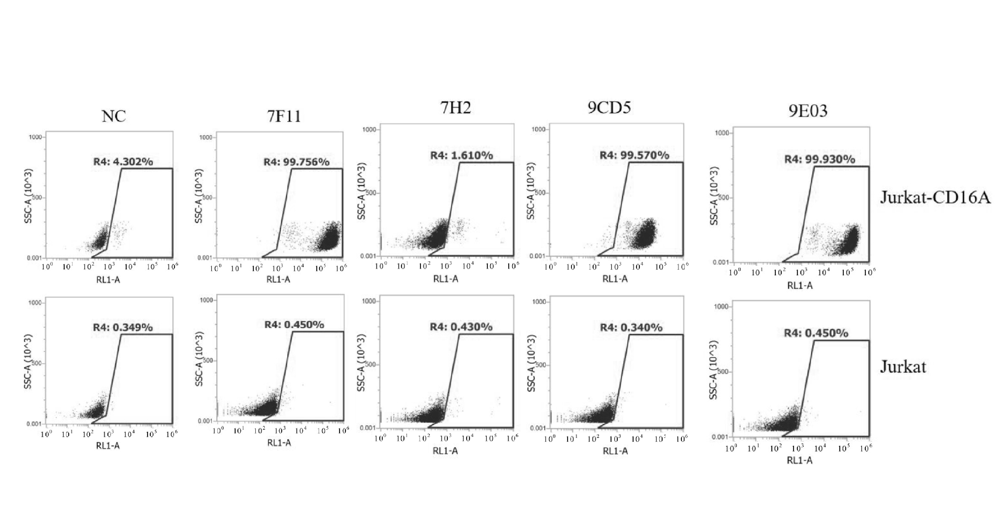

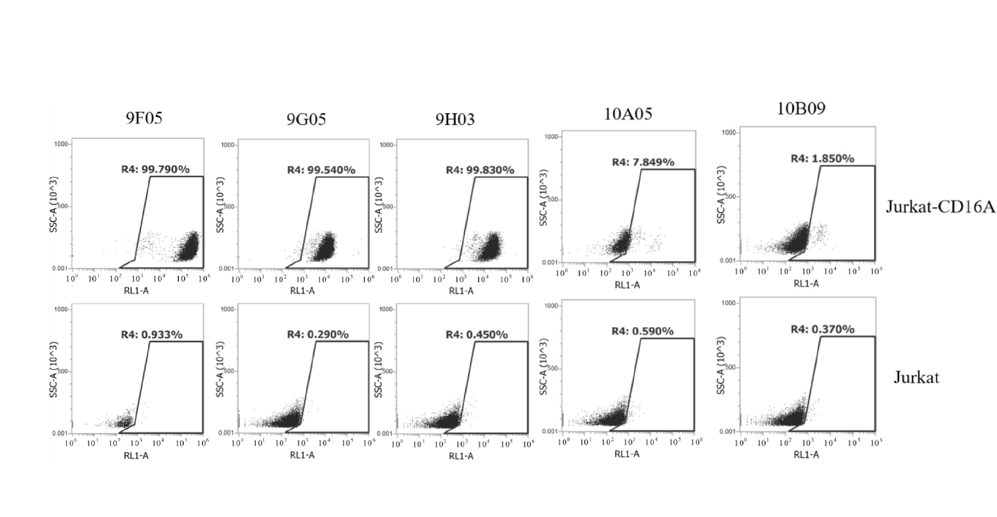

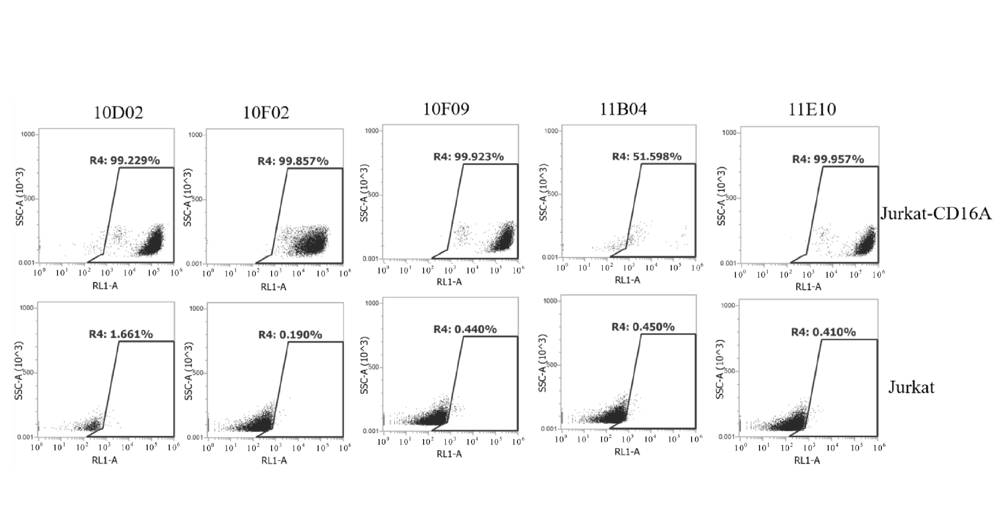

The phage ELISA results of the fourth-round output products are shown in Figure 4. Plate 1 shows OD450 values for single-clone detection on target antigen-coated plates, Plate 2 shows OD450 values on BSA-coated control plates, and Plate 3 shows S/N values (Plate 1 / Plate 2 at the same position). Clones with S/N >4 were selected for sequencing.

The candidate antibody sequences obtained from screening are as follows:
> 9-G05(SEQ ID NO: 1) 
QVKLEESGGGLVQPGGSLRLSCAASGFTFSSYAMNWVRQAPGKGLEWVSGINSG
GGYTSYADSVKGRFTISRDNAKNTLYLQMNSLKPEDTAVYYCAKYPRFYSSSYDYIPP
VRSQGTQVTVSS
>9-F05(SEQ ID NO: 2) 
QVQLVESGGGLVQAGGSLRLSCAASGRTFSRHAMGWFRQAPGKEREFVAGISWS
GSHTYYADAVKGRFTISRDNAKNTVYLQMNTLNPEDTAVYYCAASSHLSDMILIGSSD
YWGQGTQVTVSA
>9-C05(SEQ ID NO: 3) 
QVQLVESGGGLVQAGGSPRLSCAASGRTFSRHAMGWFRQAPGKEREFVAGISWS
GGSTYYADSVKGRFTISRDNAKNTVYLQMNTLNPEDTAVYYCAASSHMSDMILIGSSD
YWGQGTQVTVSA
>9-H03(SEQ ID NO: 4) 
EVQLVESGGGLVQTGGSLRLSCAASGRTFTSYNMGWFRQAPGSEREFLAAIYWN
GGPTRYADSVKGRFTISRDNAKNTVYLQMNSLKPEDTAVYYCAAAFRYNTSWNNPDF
RSWGQGTQVTVAS
>7-F11(SEQ ID NO: 5) 
AVQLVESGGGLVQAGGTLTLSCALSGRTFRSYAMGWFRQAPGKEREFVASIRWM
TGATDYTDSVKGRFAISRDNAKFTAYLQMNSLKPEDTAVYYCASASAATTWTGRAGS
YDYWGQGSQVTVSS
>7-H2(SEQ ID NO: 6)

QLQLVESGGGLVQPGGSLRLSCTASGFSLDSYVLGWFRQAPGKEREGISCITGSGG
SPTYVDSVKGRFTASIDNDRTTVYLQMNNLKPEDTAVYYCAAEFGTSICSDPRSHVGY
WGQGTQVTVSS

The CDR region sequences of the candidate antibodies are shown in Table 12.

**Table 12**

| Antibody Name | CDR1 | CDR2 | CDR3 | VHH Chain Amino Acid Sequence No. |
|---|---|---|---|---|
| 9-G05 | GFTFSSYA | INSGGGYT | AKYPRFYSSSYDYIPPV | 1 |
| 9-F05 | GRTFSRHA | ISWSGSHT | AASSHLSDMILIGSSDY | 2 |
| 9-C05 | GRTFSRHA | ISWSGGST | AASSHMSDMILIGSSDY | 3 |
| 9-H03 | GRTFTSYN | IYWNGGPT | AAAFRYNTSWNNPDFRS | 4 |
| 7-F11 | GRTFRSYA | IRWMTGAT | ASASAATTWTGRAGSYDY | 5 |
| 7-H2 | GFSLDSYV | ITGSGGSP | AAEFGTSICSDPRSHVGY | 6 |

### 1.9 Amplification of Overlap PCR Products, Transient Transfection, and Detection

### (1) First-round PCR: amplification of CMV, VHH, and FC

A 50 µL PCR reaction system was prepared as shown in Table 13. Primers for CMV fragment: CMV-F and PCom-R1; for VHH fragment: PCom-F1 and PCom-R2; for FC fragment: PCom-F2 and PGK-R.

**Table 13 PCR reaction system**

| Component | Amount |
|---|---|
| Forward primer (5 µM) | 2 µL |
| Reverse primer (5 µM) | 2 µL |
| NuHi Power mix (2×) | 25 µL |
| Template (plasmid or bacterial culture) | 1 µL |
| Sterile water | make up to 50 µL |

PCR program as shown in Table 14:

**Table 14 PCR program settings**

| Temperature | Time | Cycles |
|---|---|---|
| 95°C | 10 min | |
| 95°C | 15 s | 25X |
| 56°C | 30 s | |
| 68°C | 60 s | |
| 68°C | 10 min | |

50 µL of PCR product was mixed with 1/10 volume of 10× loading buffer, and analyzed by 1% agarose gel electrophoresis. The expected band sizes were about 750 bp for CMV and FC, and about 560 bp for VHH. The target bands were excised and purified, and the concentration was measured with a NanoDrop (if too concentrated, dilute for subsequent reactions).

### (2) Second-round PCR: The CMV, VHH and FC were fused by Overlap Extension PCR.

The PCR reaction system is shown in Table 15.

**Table 15 Second-round PCR system**

| Component | Amount |
|---|---|
| CMV 1st product | 50 ng |
| VHH 1st product | 50 ng |
| FC 1st product | 50 ng |
| NuHi Power mix (2×) | 25 µL |
| Sterile water | make up to 46 µL |

PCR program as shown in Table 16:

**Table 16 PCR program**

| Temperature | Time | Cycles |
|---|---|---|
| 95°C | 10 min | |
| 95°C | 15 s | 15X |
| 60°C | 30 s | |
| 68°C | 120 s | |

The primers CMV-F and PGK-R were added at 2 µL each. PCR program as shown in Table 17:

**Table 17 PCR program**

| Temperature | Time | Cycles |
|---|---|---|
| 95°C | 10 min | |
| 95°C | 15 s | 25X |
| 56°C | 30 s | |
| 68°C | 120 s | |
| 68°C | 10 min | |

The overlap PCR product was purified using Takara's DNA fragment purification kit, and the concentration was measured with a NanoDrop. At least 10 µg of PCR product was required for subsequent cell transfection validation. The PCR product was transiently transfected into 293F cells, and the supernatant was collected for FACS detection.

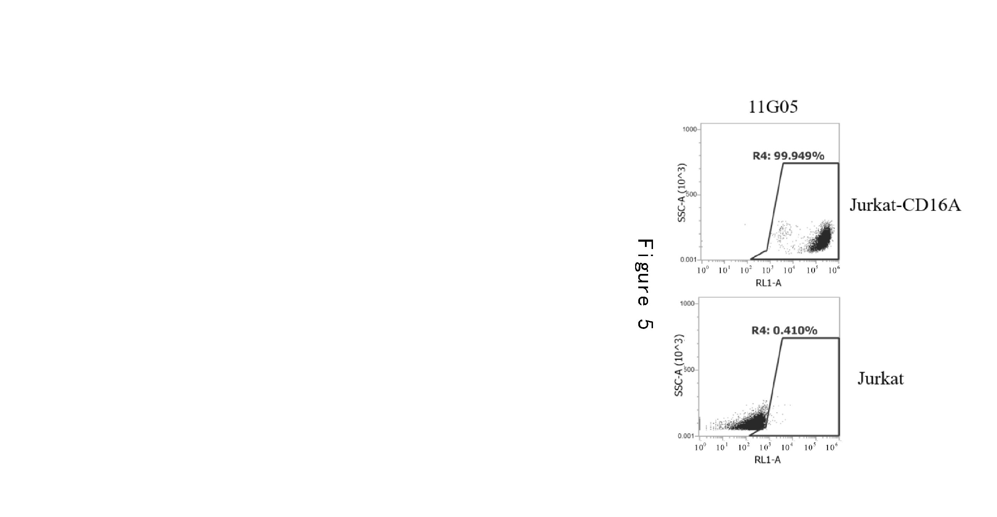

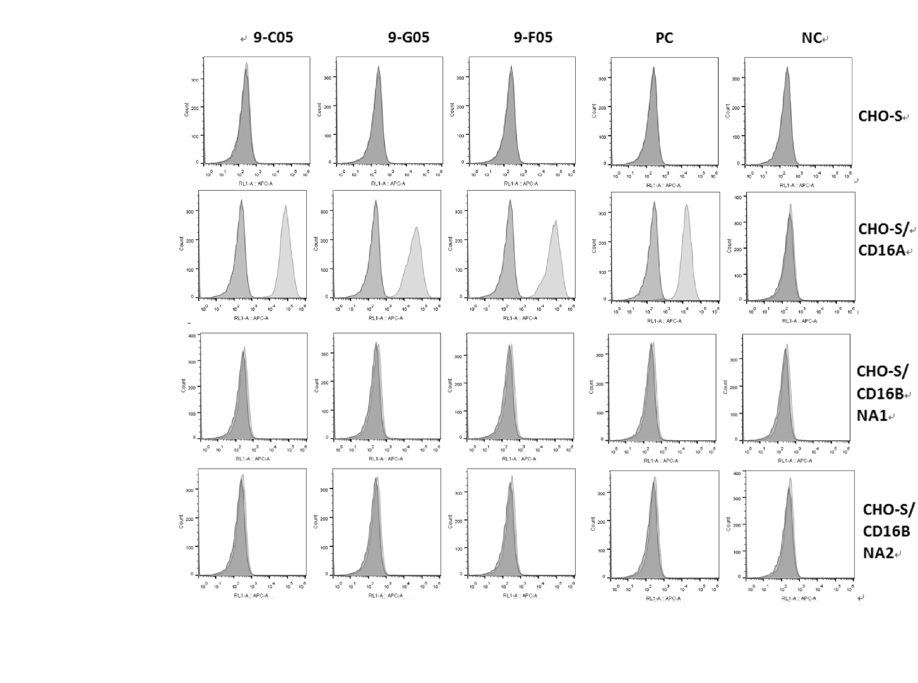

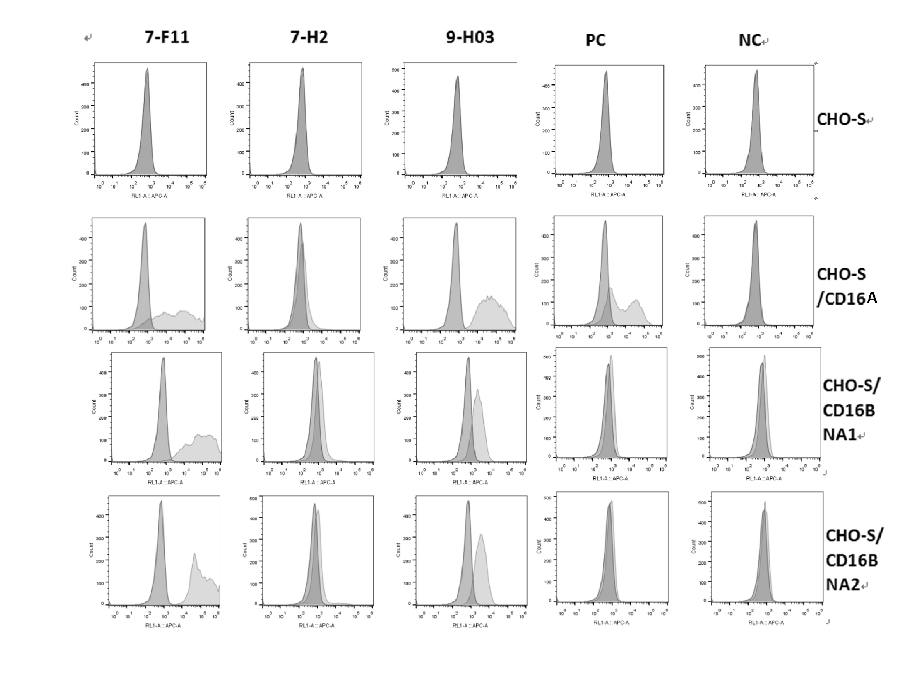

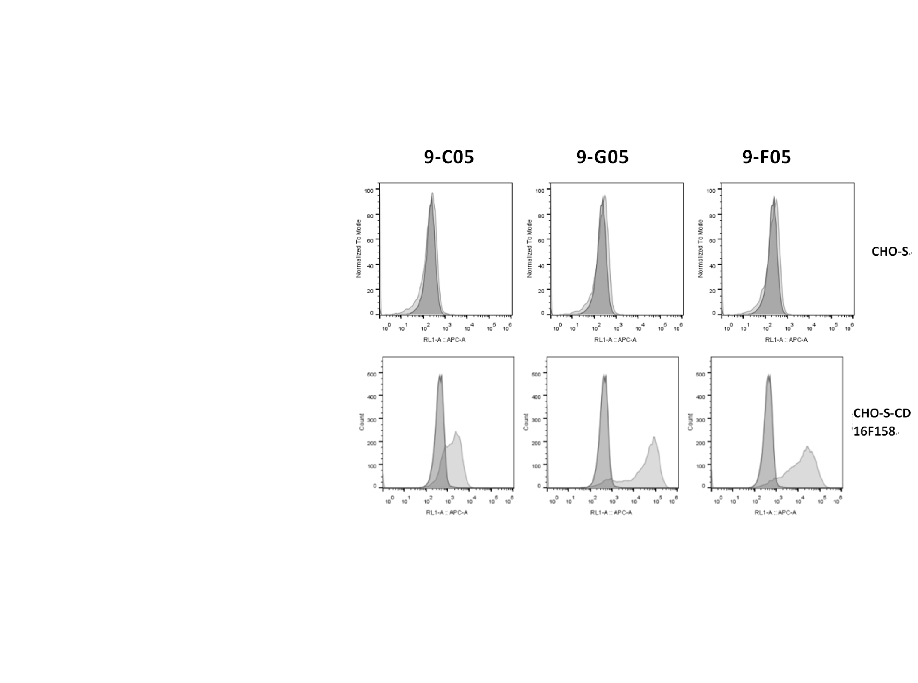

The FACS results are shown in Figure 5. Clones 7-F11, 9-F05, 9-G05, 9-H03, etc., could bind to CD16A-expressing Jurkat, but not to Jurkat cells that do not express CD16A.

### 1.10 FACS Detection of Specific Binding of Recombinant Antibodies to Target Protein

Based on FACS screening, positive clones were selected for antibody production, and the prepared antibodies were subjected to FACS binding validation. The experimental method was as follows:
CHO, CHO-CD16A, and CHO-CD16B (NA1 and NA2 subtypes) cell lines were thawed from liquid nitrogen and adjusted to logarithmic growth phase. Each cell line was divided into several aliquots, each containing 3×10⁵ cells. The expressed antibodies were incubated with the target cells, mixed well, and incubated at room temperature for 1 hour. After centrifugation at 800×g for 5 min at room temperature, the supernatant containing the antibody was removed, and the cells were washed three times with PBS. 100 µL of PE-labeled Anti-human IgG (1:500 dilution) was added, mixed well, and incubated at room temperature in the dark for 30 minutes. After centrifugation at 800×g for 5 min, the supernatant containing the secondary antibody was discarded, and the cells were washed three times with PBS. The cells were resuspended in 500 µL of PBS for flow cytometric analysis.

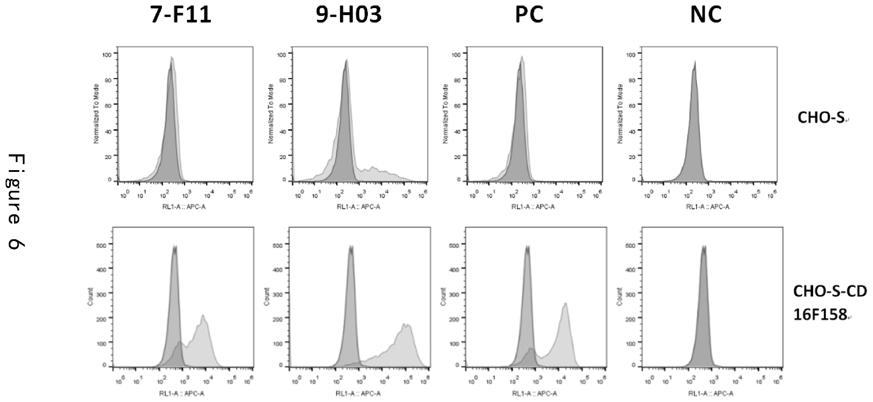

The results are shown in Figure 6. Antibodies 9-C05, 9-G05, 9-F05, 7-F11, 9-H03, etc., all bound to CHO cells expressing CD16A, but not to CHO cells expressing CD16B NA1 or CD16B NA2, demonstrating the specific binding of the antibodies of the present invention to the target protein.

### 1.11 Affinity Measurement

The affinities of antibodies 9-F05, 9-C05, and 9-G05 were measured. The experimental method was as follows: SA sensors were used to immobilize Biotin-CD16A-176v-His at a concentration of 5 µg/mL for 60 s. The buffer was PBST (PBS + 0.02% Tween 20). The antibodies PCDNA3.4-SDAB210108-9F05-His, PCDNA3.4-SDAB210108-9C05-His, and PCDNA3.4-SDAB210108-9G05-His were diluted to 50, 25, 12.5, 6.25, 3.13, and 0 nM. Affinity measurement: equilibration for 60 s, association for 180 s, dissociation for 180 s, at 25°C.

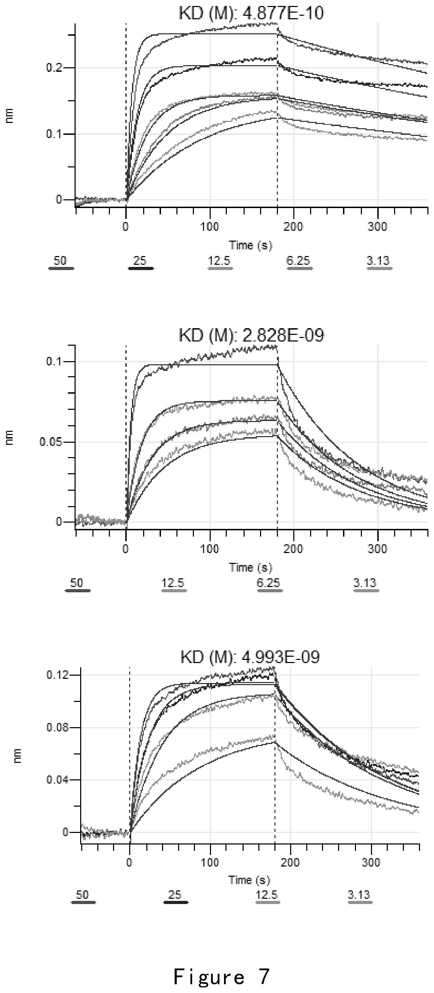

The results are shown in Figure 7 and Table 18. All three antibodies, 9-F05, 9-C05, and 9-G05, showed very high affinity.

**Table 18 Affinity measurement results of antibodies**

| Antibody Name | KD (M) | Kon (M⁻¹s⁻¹) | Koff (s⁻¹) |
|---|---|---|---|
| 9-F05 | 4.877×10⁻¹⁰ | 3.065×10⁶ | 1.495×10⁻³ |
| 9-C05 | 2.828×10⁻⁹ | 3.650×10⁶ | 1.032×10⁻² |
| 9-G05 | 4.993×10⁻⁹ | 1.428×10⁶ | 7.131×10⁻³ |

All documents mentioned in the present invention are hereby incorporated by reference into this application, as if each document were individually incorporated by reference. Furthermore, it should be understood that, after reading the above teaching of the present invention, those skilled in the art may make various changes or modifications to the present invention, and such equivalents shall also fall within the scope defined by the appended claims of this application.

## Claims

1. An anti-CD16A nanobody, wherein the anti-CD16A nanobody has one or more complementarity determining regions (CDRs) selected from the group consisting of:
(1) CDR1 as shown in SEQ ID NO: 7, CDR2 as shown in SEQ ID NO: 8, and CDR3 as shown in SEQ ID NO: 9;
(2) CDR1 as shown in SEQ ID NO: 10, CDR2 as shown in SEQ ID NO: 11, and CDR3 as shown in SEQ ID NO: 12;
(3) CDR1 as shown in SEQ ID NO: 10, CDR2 as shown in SEQ ID NO: 13, and CDR3 as shown in SEQ ID NO: 14;
(4) CDR1 as shown in SEQ ID NO: 15, CDR2 as shown in SEQ ID NO: 16, and CDR3 as shown in SEQ ID NO: 17;
(5) CDR1 as shown in SEQ ID NO: 18, CDR2 as shown in SEQ ID NO: 19, and CDR3 as shown in SEQ ID NO: 20;
(6) CDR1 as shown in SEQ ID NO: 21, CDR2 as shown in SEQ ID NO: 22, and CDR3 as shown in SEQ ID NO: 23.

2. The anti-CD16A nanobody of claim 1, wherein the CDR1, CDR2 and CDR3 are separated by framework regions FR1, FR2, FR3 and FR4 of the VHH chain.

3. The anti-CD16A nanobody of claim 1, wherein the amino acid sequence of the VHH chain of the anti-CD16A nanobody is as shown in any one of SEQ ID NOs: 1-6.

4. The anti-CD16A nanobody of claim 1, wherein the anti-CD16A nanobody comprises a humanized antibody, an animal-derived antibody, or a chimeric antibody.

5. An anti-CD16A multivalent antibody or an antibody directed against multiple epitopes, wherein the multivalent antibody or antibody directed against multiple epitopes comprises at least one antibody element directed against a CD16A epitope, wherein the antibody element is the anti-CD16A nanobody of claim 1.

6. A recombinant protein, wherein the recombinant protein has:
(i) the anti-CD16A nanobody of claim 1, or the anti-CD16A multivalent antibody or antibody directed against multiple epitopes of claim 5; and
(ii) optionally a tag sequence that assists expression and/or purification.

7. An antibody-drug conjugate, wherein the antibody-drug conjugate comprises:
(a) the nanobody of claim 1, the multivalent antibody or antibody directed against multiple epitopes of claim 5, or the recombinant protein of claim 6; and
(b) a conjugated moiety conjugated to the antibody moiety, wherein the conjugated moiety is selected from the group consisting of: detectable labels, drugs, toxins, cytokines, radionuclides, enzymes, and combinations thereof.

8. A chimeric antigen receptor (CAR), wherein the CAR comprises an extracellular domain, and the extracellular domain comprises the anti-CD16A nanobody of claim 1, or the anti-CD16A multivalent antibody of claim 5.

9. A polynucleotide, wherein the polynucleotide encodes a protein selected from the group consisting of: the nanobody of claim 1, the multivalent antibody or antibody directed against multiple epitopes of claim 5, the recombinant protein of claim 6, and the CAR of claim 8.

10. An expression vector, wherein the expression vector comprises the polynucleotide of claim 9.

11. A host cell, wherein the host cell comprises the expression vector of claim 10, or has integrated into its genome the polynucleotide of claim 9, or expresses the nanobody of claim 1, the multivalent antibody or antibody directed against multiple epitopes of claim 5, the recombinant protein of claim 6, or the CAR of claim 8.

12. A pharmaceutical composition, wherein the pharmaceutical composition comprises:
(i) the nanobody of claim 1, the multivalent antibody or antibody directed against multiple epitopes of claim 5, the recombinant protein of claim 6, the antibody-drug conjugate of claim 7, or the host cell of claim 11; and
(ii) a pharmaceutically acceptable carrier.

13. Use of the nanobody of claim 1, the multivalent antibody or antibody directed against multiple epitopes of claim 5, the recombinant protein of claim 6, the antibody-drug conjugate of claim 7, or immune cells expressing the CAR of claim 8, in the preparation of a medicament for the prevention and/or treatment of CD16A-associated cancer or disease.

14. The use of claim 13, wherein the CD16A-associated disease or disorder is selected from the group consisting of: hematological tumors, solid tumors, and combinations thereof.

15. A method for treating a disease, wherein the method comprises administering to a subject in need thereof: the nanobody of claim 1, the multivalent antibody or antibody directed against multiple epitopes of claim 5, the recombinant protein of claim 6, the antibody-drug conjugate of claim 7, or immune cells expressing the CAR of claim 8.
